# EUROPÄISCHE PATENTSCHRIFT

(11) **EP 4 324 706 B1**
(45) Veröffentlichungstag und Bekanntmachung des Hinweises auf die Patenterteilung: **07.10.2026**
(21) Anmeldenummer: 23189612.7
(22) Anmeldetag: 03.08.2023
(51) Int. Cl.: B60R 16/023, G07C 5/00, G07C 5/08, A61B 5/18, G01S 13/02, G08B 21/02, G08B 21/22, H04B 1/3822

(54) **VERFAHREN ZUM ERMITTELN VON POSITION UND VITALPARAMETER EINES INSASSEN EINES KRAFTFAHRZEUGS UND KRAFTFAHRZEUG**
METHOD FOR DETERMINING THE POSITION AND VITAL PARAMETERS OF AN OCCUPANT OF A MOTOR VEHICLE, AND MOTOR VEHICLE
PROCÉDÉ DE DÉTERMINATION DE LA POSITION ET DES SIGNES VITAUX D'UN OCCUPANT D'UN VÉHICULE AUTOMOBILE ET VÉHICULE AUTOMOBILE

(30) Priorität: 15.08.2022 DE 102022208460
(43) Veröffentlichungstag der Anmeldung: 21.02.2024
(73) Patentinhaber: VOLKSWAGEN AG, 38440 Wolfsburg (DE)
(72) Erfinder: Ette, Bernd, 38442 Wolfsburg (DE); Sacher, Patrick, 38465 Brome (DE)

(56) Entgegenhaltungen:
- DE-A1- 102021 206 343
- US-A1- 2003 184 065
- US-A1- 2016 275 790
- US-A1- 2020 348 406

## Beschreibung

Die Erfindung betrifft ein Verfahren zum Ermitteln von Position und Vitalparameter eines Insassen eines Kraftfahrzeugs und ein Kraftfahrzeug mit einem Funksystem und einer mit dem Funksystem verbundenen Steuereinheit, die zur Durchführung des Verfahrens unter Verwendung des Funksystems eingerichtet ist.

Die Sicherheit von Insassen in Fahrzeugen ist seit Langem Gegenstand moderner Entwicklung. So umfassen moderne Fahrzeuge eine Vielzahl von Sicherheitssystemen, welche das Auftreten von Unfällen oder deren Unfallfolgen reduzieren sollen. Ein klassisches Beispiel ist ein Airbag. Der Airbag wird mittels sitzplatzaufgelösten Informationen von Drucksensoren in den Fahrzeugsitzen und über erfasste Sensorwerte von in dem Fahrzeug verbauten Inertialsensoren aktiviert, um den Insassen im Falle eines Unfalls vor schwerwiegenden körperlichen Schäden möglichst zu bewahren.

Allerdings können Airbags auch ungewollt auslösen. So ist es allein auf Grundlage der Drucksensorwerte schwierig, zwischen einem Gegenstand auf dem Sitzplatz und einem sitzenden Insassen zu unterscheiden. Daher ist es offensichtlich, dass es einer Innenraum-Sensorik bedarf, die einen Insassen von einem Gegenstand unterscheiden kann und die Position des Insassen möglichst genau in dem Fahrzeug ermitteln kann, um den Sicherheitssystemen des Fahrzeugs eine möglichst präzise Grundlage zur Erfüllung ihres vorhergesehenen Zwecks zu ermöglichen.

In dem Dokument WO 2021220190 A1 wird ein Verfahren und ein System zur Überwachung einer Fahrzeugkabine vorgeschlagen, welches ein Radar-Sensor-Array-System im Dachhimmel des Fahrzeugs vorschlägt, welches reflektierte Signale von Insassen in dem Fahrzeug erfasst. Mittels einer Datenanalyse werden die Größe des Insassen, seine Position, seine Bewegungen und seine Vitalzeichen ermittelt. So ist es mit nur einer einzigen Sensoranordnung möglich, ein multifunktionales Überwachen der Fahrzeugkabine durchzuführen und eine Vielzahl von dafür sonst benötigten Sensoren zu ersetzen.

Dokument DE 10 2020 124 444 A1 bezieht sich auf ein Verfahren und eine Überwachungsvorrichtung zum Überwachen eines Innenraums und/oder eines Außenbereichs eines Kraftfahrzeugs. Hierzu wird zunächst eine elektromagnetische Strahlung in den Innenraum und/oder Außenbereich ausgestrahlt und die an einem Objekt reflektierte elektromagnetische Strahlung empfangen, um daraus eine Information über einen Zustand des Objekts abzuleiten.

Weitere Beispiele zur Positionsbestimmung unter Verwendung von elektromagnetischer Strahlung werden in den Dokumenten DE 10 2021 206 343 A1, DE 10 2020 215 852 A1, US 2003/0184065 A1 und US 2016/0275790 A1 beschrieben.

Der Erfindung liegt nun die Aufgabe zugrunde, ein alternatives Verfahren zum Ermitteln einer Position und eines Vitalparameters eines Fahrzeuginsassen zu entwickeln.

Die erfindungsgemäße Aufgabe wird gelöst durch ein Verfahren zum Ermitteln von Position und Vitalparameter eines Insassen eines Kraftfahrzeugs und ein Kraftfahrzeug gemäß den unabhängigen Ansprüchen. Bevorzugte Weiterbildungen sind Gegenstand der jeweils rückbezogenen Unteransprüche.

Ein erster Aspekt betrifft ein Verfahren zum Ermitteln von Position und Vitalparameter eines Insassen eines Kraftfahrzeugs. Das Kraftfahrzeug umfasst ein Funksystem mit einem Sendeempfänger, mit einer ersten Ultra-Wide-Band(UWB)-Antenne und mit einer zweiten UWB-Antenne und eine mit dem Funksystem verbundene Steuereinheit. Die erste UWB-Antenne und die zweite UWB-Antenne sind zum Senden und Empfangen von UWB-Pulsen eingerichtet. Die Steuereinheit ist dazu eingerichtet, das Funksystem zum Durchführen eines auf Channel Impulse Response(CIR)-Messungen basierenden Verfahrens unter Verwendung der ersten UWB-Antenne und der zweiten UWB-Antenne während des Betriebs des Kraftfahrzeugs anzusteuern und die Position und einen Vitalparameter des Insassen basierend auf einem Ergebnis der CIR-Messungen zu ermitteln. Vitalparameter sind Maßzahlen, die Grundfunktionen des menschlichen Körpers widerspiegeln und zur Überprüfung der Vitalfunktionen gemessen werden können. Darunter fallen beispielweise die Herzfrequenz, der Blutdruck, die Körpertemperatur und/oder die Atemfrequenz. Betrieb des Kraftfahrzeugs bedeutet während einer Fahrt mit dem Kraftfahrzeug und/oder wenn die Zündung des Kraftfahrzeugs eingeschaltet ist.

Der Sendeempfänger ist insbesondere zum Senden und Empfangen von Signalen in sehr großen Frequenzbereichen, insbesondere in einem Frequenzbereich von 3,1 GHz bis 10,6 GHz, bevorzugt in einem Frequenzbereich von 3,5 GHz bis 9 GHz, besonders bevorzugt in einem Frequenzbereich von 6 GHz bis 8,5 GHz, ausgebildet. Die Sendeleistung der UWB-Pulse ist dabei gering. Die Bandbreite des UWB-Signals beträgt mindestens 500 MHz und der UWB-Sendeempfänger ist bevorzugt zum Senden von Signalen mit einer Sendeleistung zwischen 0,5 mW / -41,3 dBm/MHz ausgebildet. Ferner bevorzugt ist der Sendeempfänger gemäß dem Standard IEEE 802.15.4 (insbesondere den Abschnitten zum UWB PHY Layer) und bevorzugt gemäß dem Standard IEEE 802.15.4z ausgebildet. Durch die Streuung der Signale über derart große Frequenzbereiche stören UWB-Signale andere Funksignale nur minimal.

Aufgrund der zeitlich stark lokalisierten UWB-Pulse ist es möglich, aus empfangenen UWB-Pulsen mit deren sich aus dem Einfluss der Umgebung auf den gesendeten UWB-Puls ergebender Impulsantwort Informationen zu dem Propagationsweg der UWB-Pulse zu extrahieren. Einflüsse der Umgebung basieren auf physikalischen Phänomenen, die den UWB-Puls von seinem geometrisch vorgeschriebenen Weg ablenken, wie beispielsweise Brechung, Beugung, Reflexion oder Dämpfung. Es ist offensichtlich, dass sich die Laufzeiten der Signale oder Signalpakete entlang verschiedener Propagationswege unterscheiden und in Abhängigkeit von der Anwesenheit oder Abwesenheit von Objekten in oder nahe der Propagationswege verändern. Auch die Impulsform der Signale oder Signalpakete wird in Abhängigkeit von der Anwesenheit oder Abwesenheit von Objekten in oder nahe der Propagationswege beeinflusst. Somit lässt sich anhand der Messung von entlang dieser Propagationswege übertragener Signale oder Signalpakete vorteilhaft auf die Anwesenheit oder Abwesenheit von Objekten in oder nahe der Propagationswege schließen.

Vorteilhaft ist es unter Verwendung von nur (mindestens) zwei UWB-Antennen möglich, die Umgebung mittels der CIR-Messungen ortsaufgelöst abzutasten. Durch mehrfaches Versenden von UWB-Pulsen über eine der Antennen und anhand der durch die andere Antenne empfangenen Impulsantworten können Veränderungen in der Umgebung - beispielsweise ein neu in dem abzutastenden Bereich befindliches Objekt - durch den Vergleich der zeitversetzten Impulsantworten orts- und zeitaufgelöst sichtbar gemacht werden. Auf diese Weise ist die Detektion eines in den abzutastenden Bereich eintretenden Objektes verlässlich möglich. Insbesondere ein besonders bevorzugtes konstantes und/oder repetitives Abtasten der Umgebung mittels CIR-Messungen erlaubt dabei eine entsprechende Überwachung des abzutastenden Bereichs.

Die CIR-Messungen beinhalten beispielsweise das Versenden von vordefinierten Signalen oder Signalpaketen (sogenannten Telegrammen) zwischen den (zumindest) zwei UWB-Antennen. Neben einem direkten Propagationsweg des Signals oder der Signalpakete zwischen den UWB-Antennen gibt es eine Vielzahl weiterer Propagationswege, die Reflektionen von Objekten aus dem Innen- oder Außenraum des Fahrzeugs einschließen. Basierend auf einer ausreichenden Anzahl und/oder einer vorteilhaften Anordnung der UWB-Antennen ermöglichen diese Propagationswege eine Abdeckung von weiten Teilen eines Raums, beispielsweise eines Fahrzeuginnenraums oder eines Fahrzeugaußenraums. Selbstverständlich können die CIR-Messungen zwischen einer Vielzahl von UWB-Antennen erfolgen, um abhängig von der Anordnung der UWB-Antennen entsprechende Bereiche, insbesondere einen Innenraum eines Kraftfahrzeugs, abzutasten.

Vorteilhaft werden die UWB-Antennen so angeordnet, dass sich ein oder mehrere Insassen in dem Fahrzeug sitzplatzgenau oder -aufgelöst erkennen lassen. Insbesondere erfolgt eine Erkennung der Sitzplatzbelegung des Fahrzeugs während des Fahrzeugbetriebs. Zudem lassen sich aus den CIR-Messungen Bewegungen des/der Insassen, insbesondere die Brustbewegung einer Atmung des/der Insassen, auflösen. Bevorzugt sind die beiden UWB-Antennen in Türen, vorzugsweise in gegenüberliegenden Türen, des Fahrzeugs angeordnet. Beispielsweise kann die erste UWB-Antenne in der Fahrertür und die die zweite UWB-Antenne in der Beifahrertür des Fahrzeugs angeordnet sein. Hierdurch können die beiden vorderen Plätze in einem Fahrzeug mittels CIR-Messungen überwacht werden. Selbstverständlich können die UWB-Antennen analog oder zusätzliche UWB-Antennen in den hinteren beiden gegenüberliegenden Türen des Fahrzeugs angeordnet sein, um die hinteren Sitzplätze zu überwachen. Es ist aber auch eine beliebig andere Anordnung der UWB-Antennen möglich. Bevorzugt ist eine Vielzahl von UWB-Antennen vorgesehen. Dies hat den Vorteil, dass eine Vielzahl von Insassen, deren Position und Vitalparameter basierend auf den CIR-Messungen erkannt und überwacht werden können. Ferner vorteilhaft können bereits im Fahrzeug verbaute UWB-Antennen für das erfindungsgemäße Verfahren benutzt werden. Moderne Fahrzeuge weisen teilweise UWB-Antennen auf, die in dem Fahrzeug zwecks schlüssellosem Zugangs verbaut sind. Folglich werden die bereits verbauten UWB-Antennen multifunktional verwendet und Kosten gespart werden. Ferner hat die zusätzliche Nutzung von bereits verbauten UWB-Antennen den Vorteil, dass (teilweise) redundante Antennen vorhanden sind. Insbesondere in einem Unfallszenario können einzelne UWB-Antenne defekt sein, aber durch redundante Antennen aufgefangen werden. Insofern können die CIR-Messungen trotz teilweise defekter UWB-Antennen weiterhin durchgeführt werden.

Die ermittelten Positions- und Bewegungsdaten und der ermittelte Vitalparameter des Insassen werden bevorzugt in einer Speichereinheit des Kraftfahrzeugs hinterlegt. Durch das Abspeichern der Positions- und Bewegungsdaten und der ermittelten Vitalparameter des Insassen können insassenindividuelle Profile erstellt werden, auf welche in zukünftigen Situationen zurückgegriffen werden kann, um eine individuelle und damit bessere Entscheidung im Einzelfall treffen zu können.

Ferner ist vorgesehen, dass ein Unfall des Kraftfahrzeugs erfasst wird. Nach dem (erfassten) Unfall erfolgt ein erneutes Ansteuern des Funksystems zum erneuten Durchführen des auf CIR-Messungen basierenden Verfahrens unter Verwendung der ersten UWB-Antenne und der zweiten UWB-Antenne. Die Position und der Vitalparameter des Insassen wird erneut basierend auf einem Ergebnis der erneuten CIR-Messungen ermittelt. Anhand der Veränderungen in der Position und den Vitalparameter des Insassen zwischen vor und nach dem Unfall lassen sich Informationen zu den Einwirkungen des Unfalls auf Insassen ableiten. Kenntnisse über Anzahl, Position, Gesundheitszustand (Vitalparameter), Körpergröße (Erwachsener, Kind, Baby) des oder der Insassen können heraneilende Rettungsteams die Rettung und Bergung des oder der Insassen wesentlich erleichtern und somit die Unfallfolgen reduzieren. Selbstverständlich können auch nach dem Unfall eine Vielzahl von, insbesondere so viele CIR-Messungen wie möglich oder nötig durchgeführt werden, bis beispielsweise kein Insasse in dem Fahrzeug mehr ermittelt wird.

Vorzugsweise werden die ermittelten Positions- und Bewegungsdaten und der ermittelte Vitalparameter des oder der Insassen nach dem Unfall in der Speichereinheit des Kraftfahrzeugs hinterlegt. Ebenfalls bevorzugt wird nach einem Unfall ein Notmodus des Fahrzeugs aktiviert. Im Notmodus ist das Kraftfahrzeug eingerichtet, einen Zugriff auf vorbestimmte Fahrzeugfunktionen von außen zuzulassen und entsprechende Signale an die Fahrzeugkomponenten auszugeben, um die vorbestimmten Fahrzeugfunktionen durchzuführen. Beispielsweise können Aktoren angesteuert werden, die eine Entriegelung der Zentralverriegelung oder ein Öffnen von Türen oder Fenstern steuern. Diese Aktoren sind meist nur vom Innenraum des Fahrzeugs erreichbar und somit für die Rettungsteams umso schwerer zu betätigen, um Insassen aus dem Fahrzeug zu befreien. Bevorzugt ist die Bedienungsmöglichkeit der Aktoren in dem Notmodus unabhängig von einem Zündungszustand des Fahrzeugs.

Bevorzugt erfolgt eine sitzplatzaufgelöste Erkennung des oder der Insassen des Kraftfahrzeugs basierend auf der ermittelten Position des oder der Insassen vor und nach dem Unfall. Hierfür kann beispielsweise die Sitzplatzbelegung des Fahrersitzes, des Beifahrersitzes und/oder der Fonds-Plätze herangezogen werden. Die Informationen über die Zuordnung der Insassen zu den einzelnen Sitzplätzen erleichtert es den Rettungsteams, geeignete Rettungsmaßnahmen durchzuführen, um den oder die Insassen zu befreien. So kann ein gewaltsamer Zugang zu dem Fahrzeug über eine Fensterscheibe oder eine Fahrzeugtür dort erfolgen, wo kein Insasse ermittelt wurde, um die Verletzungsgefahr bei der Rettung der Insassen zu verringern.

Ebenfalls werden die ermittelte Position und/oder der ermittelte Vitalparameter des Insassen vor dem Unfall mit der ermittelten Position und/oder dem ermittelten Vitalparameter des Insassen nach dem Unfall verglichen. Hierdurch kann vorteilhaft festgestellt werden, ob und wie ein Insasse seine Position durch den Unfall verändert hat, nämlich ob ein Insasse durch den Unfall aus dem Fahrzeug geschleudert wurde und die Rettungsteams deshalb außerhalb des Fahrzeugs noch nach dem oder nach einem weiteren Insassen suchen sollten. Ferner bevorzugt erfolgt eine Lebewesens- und Gesundheitszustandserkennung anhand der ermittelten Vitalparameter vor und nach dem Unfall. So kann individuell für jeden Insassen überprüft werden, in welchem Norm-Bereich seine Vitalparameter liegen und ob die nach dem Unfall festgestellten Vitalparameter von dem Norm-Bereich abweichen und daher auf einen verschlechterten Gesundheitszustand schließen lassen. In Kombination mit den ermittelten Vitalparametern und den ermittelten Positionen des/der Insassen kann eine Rettungsreihenfolge der Insassen festgelegt werden, um Schwerverletzte vor weniger Verletzten Insassen zu befreien. Ferner erfolgt bevorzugt eine Erkennung der Ausstattung des Fahrzeugs, um diese Informationen den die Rettungsteams bereitstellen zu können.

Ebenfalls bevorzugt wird das Funksystem basierend auf einem Ergebnis des Vergleichens angesteuert, ein Notfallsignal in die Fahrzeugumgebung auszusenden. Das Notfallsignal umfasst dabei die ermittelten Positionen und/oder die ermittelten Vitalparameter des Insassen vor und nach dem Unfall. Das Notfallsignal umfasst besonders bevorzugt ferner die Informationen über die Ausstattung des Fahrzeugs. Durch das Aussenden des Notfallsignals über das Funksystem können andere Fahrzeuge oder Rettungseinrichtungen in der Fahrzeugumgebung über die Notlage und detaillierten Informationen zu den Insassen und dem Fahrzeug informiert werden, um einen Rettungseinsatz zu initiieren.

Ebenfalls bevorzugt sind die erste UWB-Antenne und die zweite UWB-Antenne ferner zum Senden und Empfangen von Bluetooth-Funksignalen eingerichtet und das Funksystem wird angesteuert, das Notfallsignal als UWB- und/oder Bluetooth-Funksignal unter Verwendung der ersten UWB-Antenne und/oder zweiten Antenne auszusenden. Die Bluetooth-Kommunikation erfolgt bevorzugt in dem bekannten Frequenzband zwischen 2,402 GHz und 2,480 GHz. Bevorzugt ist die Bluetooth-Low-Energy-Funktechnologie vorgesehen. Gegenüber bekannten Lösungen wird hiermit eine Kommunikationstechnologie verwendet, die ohne eine aktive WLAN-, GPS- oder LTE-Funktion direkt vor Ort durch das Fahrzeug zur Verfügung gestellt werden kann. Nach einem Unfall ist bei den bekannten Lösungen nicht sichergestellt, dass das Notfallsignal rechtzeitig an eine Leitstelle übertragen werden konnte. Technische Probleme der Kommunikationssysteme des Fahrzeugs können nach einem Unfall nicht ausgeschlossen werden. Auch die Qualität und Verfügbarkeit der Netzwerkverbindungen sind nicht verlässlich einschätzbar. Demnach kann auch in diesen Fällen das Notfallsignal zumindest von mobilen Endgeräten von Personen oder dem Rettungsteam in der Fahrzeugumgebung empfangen werden. Bevorzugt werden die Antennen angesteuert, die Notfallsignale zeitversetzt auszusenden, um Energie zu sparen und die Sendedauer zu erhöhen. Beispielsweise erfolgt das Aussenden des Notfallsignals alle zehn Sekunden.

Ebenfalls bevorzugt umfasst das Notfallsignal allgemeine Informationen über das Kraftfahrzeug. Beispielsweise können diese Informationen den Umstand betreffen, ob es sich um ein Elektro-Auto oder ein gasbetriebenes Auto handelt, von denen eine erhöhte Brandgefahr ausgehen kann. Außerdem können die allgemeinen Informationen die Anordnung und den Auslösezustand der Airbags des Fahrzeugs oder die Punkte an der Fahrzeugkarosserie, an denen eine Schere zum Öffnen des Fahrzeugs sicher eingesetzt werden kann, umfassen.

Ebenfalls bevorzugt umfasst das Funksystem ferner eine NFC-Antenne zum Übertragen des Notfallsignals, wobei nach einem Unfall die für das Notfallsignal erforderlichen Informationen in einem NFC-Speicher hinterlegt werden. Bevorzugt umfasst das Funksystem den NFC-Speicher. Bevorzugt ist die NFC-Antenne als NFC-Reader oder NFC-Tag ausgebildet. Ein NFC-Reader ist eine aktive Komponente bei NFC-Übermittlungen. Er ist in der Lage, NFC-Tags zu lesen und zu beschreiben und kann direkt mit anderen Geräten kommunizieren. Ein NFC-Tag hingegen ist von einer Energieversorgung eines anderen NFC-Geräts abhängig und ist in der Lage, auf Basis der Energieversorgung des anderen NFC-Geräts Informationen aus dem NFC-Speicher mittels NFC zu übertragen. Vorteilhaft können Rettungsteams mittels eines NFC-fähigen Endgeräts über die NFC-Antenne das Notfallsignal und die ermittelten Positionen und Vitalparameter des oder der Insassen erhalten. Im Falle des NFC-Tags funktioniert dies selbst nachdem die Stromversorgung des Fahrzeugs zusammengebrochen ist.

In weiterer bevorzugter Ausgestaltung der Erfindung ist vorgesehen, dass das Funksystem zum Senden von UWB-Pulsen und Empfangen von Impulsantworten unter Verwendung von mindestens einer der beiden UWB-Antennen angesteuert und die Position und/oder der Vitalparameter des oder der Insassen ferner basierend auf einem Ergebnis der empfangenden Impulsantworten ermittelt wird. Im Gegensatz zu den CIR-Messungen empfängt bei dem Verfahren mit den empfangenen Impulsantworten die gleiche UWB-Antenne, die das UWB-Signal aussendet, auch die Impulsantwort. Insofern wird hierbei ein Echo der abgetasteten Umgebung empfangen. Dieses Echo bildet für gewöhnlich eine Vielzahl von zeitlich aufgelösten Echosignalen ab, die sich in Abhängigkeit von dem Abstand dieser Objekte und/oder Personen zum Sendeempfänger in der Impulsantwort niederschlagen. Durch den Vergleich der Echosignale über zeitlich versetzte UWB-Pulse und deren empfangenen Impulsantworten können Rückschlüsse auf Ortsänderungen der Objekte und/oder Personen im Bezug zum Sendeempfänger getroffen werden. Aus den Ortsänderungen können die Vitalparameter ermittelt werden. Die Echosignale können bevorzugt anhand von Amplituden- und/oder Phaseninformationen ermittelt und miteinander verglichen werden. Dadurch ist es vorteilhaft möglich, den Innenraum des Fahrzeugs unter Verwendung von mindestens einer Antenne orts- und zeitaufgelöst abzutasten. Selbstverständlich können auch die UWB-Antennen oder andere im Fahrzeug verbaute UWB-Antennen das Verfahren der empfangenen Impulsantworten durchführen, um aus verschiedenen Winkeln die Umgebung und/oder den Innenraum des Fahrzeugs orts- und zeitaufgelöst abzutasten.

Je weiter ein Objekt von dem Sendeempfänger weg ist, desto später wird das dem Objekt zugeordnete Echosignal vom Sendeempfänger empfangen. So lässt sich eine Reichweite des Sendeempfängers beschränken, indem ein Empfangen der Impulsantwort nach einer der gewünschten Reichweite entsprechenden Zeit unterbrochen wird und/oder ein neuer UWB-Puls ausgesendet wird. In der Zeit, in welcher der UWB-Puls über den Sendeempfänger ausgesendet wird, ist kein Empfangen der Impulsantwort durch den zum Senden verwendeten Sendeempfänger möglich.

Vorteilhaft können mit dem auf den empfangenen Impulsantworten basierenden Verfahren vorhandene UWB-Antennen des Fahrzeugs genutzt werden, die üblicherweise für die Erkennung der Fahrzeugumgebung genutzt werden. Denn diese UWB-Antennen werden üblicherweise gerade nicht für das Aussenden von UWB-Signalen in den Innenraum des Fahrzeugs verwendet und stehen daher zur Verfügung. Insofern können die vorhandenen UWB-Antennen multifunktional genutzt und Kosten gespart werden. Ferner können UWB-Antennen nach einem Unfall defekt sein, sodass es von Vorteil ist, dass dieses Verfahren auch nur von lediglich einer UWB-Antenne durchgeführt werden kann. Folglich kann es insbesondere nach einem Unfall vorgesehen sein, dass das Funksystem zum Durchführen des auf Empfangen von Impulsantworten ausgesendeter UWB-Pulse basierenden Verfahrens zum Ermitteln der Position und des Vitalparameters des Insassen nach dem Unfall unter Verwendung von mindestens einer der beiden Antennen angesteuert wird.

Ein weiterer Aspekt umfasst ein Kraftfahrzeug mit einem Funksystem und einer mit dem Funksystem verbundenen Steuereinheit. Das Funksystem umfasst einen Sendeempfänger mit einer ersten UWB-Antenne und einer zweiten UWB-Antenne. Die Steuereinheit ist dazu eingerichtet, das obige durchzuführen. Die mit dem Verfahren beschriebenen Merkmale und deren Vorteile lassen sich analog mit dem Kraftfahrzeug verwirklichen und sind daher beliebig miteinander kombinierbar.

In bevorzugter Ausgestaltung der Erfindung ist vorgesehen, dass das Kraftfahrzeug ferner eine Sensoreinheit aufweist, die zusammen mit der Steuereinheit eingerichtet ist, einen Unfall des Kraftfahrzeugs zu ermitteln. Die Sensoreinheit umfasst bevorzugt einen Inertialsensor, einen Empfänger eines Airbagauslösesignals und/oder eine Kamera. Der Inertialsensor umfasst bevorzugt einen Beschleunigungs-, Schock- und/oder Überschlagssensor. Die Steuereinheit ist bevorzugt dazu eingerichtet, aus den erfassten Werten des Inertialsensors, von dem Airbagauslösesignalempfänger und/oder aus den erstellten Kamerabildern einen Unfall des Fahrzeugs zu erkennen.

Die oben genannte Steuereinheit des Kraftfahrzeugs ist bevorzugt durch elektrische oder elektronische Bauteile oder Komponenten (Hardware) oder durch Firmware (ASIC) implementiert. Zusätzlich oder alternativ wird die Funktionalität der Steuereinheit beim Ausführen eines geeigneten Programms (Software) verwirklicht. Ebenfalls bevorzugt ist die Steuereinheit durch eine Kombination von Hardware, Firmware und/oder Software verwirklicht. Beispielsweise sind einzelne Komponenten der Steuereinheit zum Bereitstellen einzelner Funktionalitäten als separat integrierter Schaltkreis ausgebildet oder auf einem gemeinsamen integrierten Schaltkreis angeordnet.

Die einzelnen Komponenten der Steuereinheit sind ferner bevorzugt als ein oder mehrere Prozesse ausgebildet, die auf einem oder mehreren Prozessoren in einem oder mehreren elektronischen Rechengeräten laufen und beim Ausführen von ein oder mehreren Computerprogrammen erzeugt werden. Die Rechengeräte sind dabei bevorzugt dazu ausgebildet, mit anderen Komponenten, beispielsweise einer Zentralverriegelung, einem Motorcontroller, et cetera zusammenzuarbeiten, um die hierin beschriebenen Funktionalitäten zu verwirklichen. Die Anweisungen der Computerprogramme sind dabei bevorzugt in einem Speicher abgelegt, wie beispielsweise einem RAM-Element. Die Computerprogramme können jedoch auch in einem nicht-flüchtigen Speichermedium, wie beispielsweise einer CD-ROM, einem Flash-Speicher oder dergleichen, abgelegt sein.

Dem Fachmann ist ferner ersichtlich, dass die Funktionalitäten von mehreren Recheneinheiten (Datenverarbeitungsgeräten) kombiniert oder in einem einzigen Gerät kombiniert sein können oder dass die Funktionalität von einem bestimmten Datenverarbeitungsgerät auf eine Vielzahl von Geräten verteilt vorliegen kann, um die Funktionalität der Steuereinheit zu verwirklichen.

Ein weiterer Aspekt der Offenbarung betrifft ein Computerprogramm, umfassend Befehle, die bei der Ausführung des Programms durch einen Computer, wie beispielsweise eine Steuereinrichtung eines Kraftfahrzeugs aufweisend eine Funksystem mit einem Sendeempfänger, aufweisend eine erste UWB-Antenne und eine zweite UWB-Antenne, diesen veranlassen, das erfindungsgemäße Verfahren, insbesondere ein Verfahren zum Ermitteln von Position und Vitalparameter eines Insassen eines Kraftfahrzeugs durchzuführen.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Darstellung eines Verfahrens gemäß einer Durchführungsform,
- Figur 2: eine schematische Darstellung eines Kraftfahrzeugs gemäß einer Ausführungsform mit fünf UWB-Antennen und
- Figur 3: eine schematische Darstellung eines Kraftfahrzeugs mit fünf UWB-Antennen gemäß einer weiteren Ausführungsform.

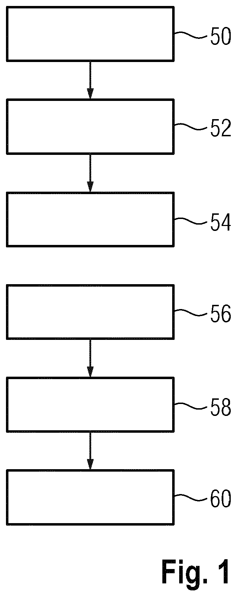

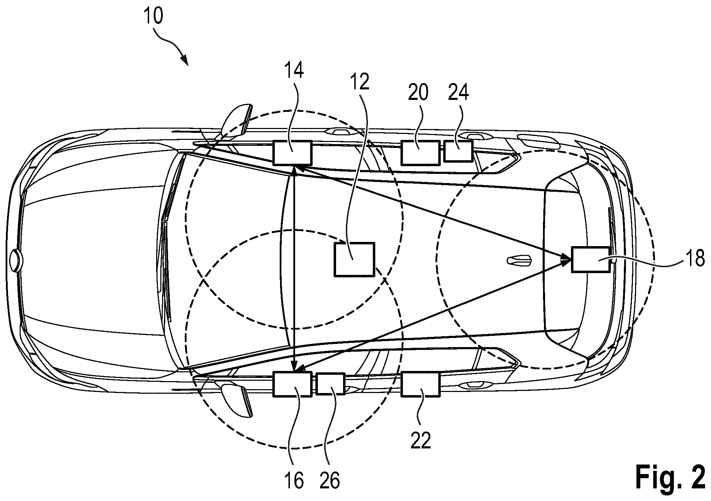

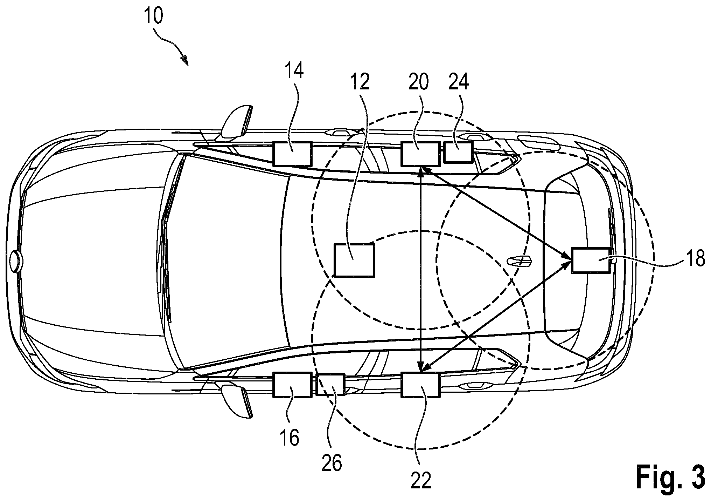

Figur 1 zeigt eine schematische Darstellung eines Verfahrens gemäß einer exemplarischen Durchführungsform. Das Verfahren ist zum Ermitteln von Position und Vitalparameter eines Insassen eines Kraftfahrzeugs 10 geeignet. Mit Blick auf die Figuren 2 und 3 wird das Kraftfahrzeug 10 anhand von zwei Ausführungsformen näher beschrieben.

Das Kraftfahrzeug 10 umfasst ein Funksystem mit einem Sendeempfänger mit mindestens einer ersten UWB-Antenne 14 und einer zweiten UWB-Antenne 16 und eine mit dem Funksystem verbundene Steuereinheit 12. Die Steuereinheit 12 ist insbesondere zur Durchführung des anhand von Figur 1 geschilderten Verfahrens eingerichtet. In den Figuren 2 und 3 wird ein exemplarisches Kraftfahrzeug 10 mit einem Funksystem, einem Sendeempfänger und einer ersten bis fünften UWB-Antenne 14, 16, 18, 20, 22 dargestellt. Die fünf UWB-Antennen 14, 16, 18, 20, 22 verteilen sich hierbei auf die fünf Türen des Kraftfahrzeugs 10. Demnach sind die erste UWB-Antenne 14 in der Beifahrertür, die zweite UWB-Antenne 16 in der Fahrertür, die dritte UWB-Antenne 18 in der Heckklappe, die vierte UWB-Antenne 20 in der Tür hinter der Beifahrertür und die fünfte UWB-Antenne 22 in der Tür hinter der Fahrertür angeordnet. Die Anzahl und Anordnung der UWB-Antennen 14, 16, 18, 20, 22 ist lediglich exemplarischer Natur, die dem besseren Verständnis dienen soll. Die Offenbarung ist nicht auf die gezeigte Anordnung und Anzahl von UWB-Antennen 14, 16, 18, 20, 22 beschränkt.

In einem ersten Verfahrensschritt 50 wird das Funksystem zum Durchführen eines auf CIR-Messungen basierenden Verfahrens unter Verwendung der ersten UWB-Antenne 14 und der zweiten UWB-Antenne 16 während des Betriebs des Kraftfahrzeugs 10 angesteuert. In Bezug auf Figur 2 ist dargestellt, dass das Funksystem zum Durchführen des auf CIR-Messungen basierenden Verfahrens unter Verwendung der ersten UWB-Antenne 14, der zweiten UWB-Antenne 16 und der dritten UWB-Antenne 18 während des Betriebs des Kraftfahrzeugs 10 angesteuert wird. In Bezug auf Figur 3 ist illustriert, dass das Funksystem zum Durchführen des auf CIR-Messungen basierenden Verfahrens unter Verwendung der vierten UWB-Antenne 20, der fünften UWB-Antenne 22 und der dritten UWB-Antenne 18 während des Betriebs des Kraftfahrzeugs 10 angesteuert wird. Offensichtlich können auch CIR-Messungen zwischen anderen, insbesondere allen, UWB-Antennen 14, 16, 18, 20, 22 erfolgen, um Informationen zu Insassen und Objekten entlang des Propagationswegs zwischen den jeweiligen UWB-Antennen 14, 16, 18, 20, 22 zu erhalten.

In einem zweiten Verfahrensschritt 52 wird eine Position und ein Vitalparameter eines Insassen des Kraftfahrzeugs 10 basierend auf einem Ergebnis der CIR-Messungen ermittelt. In diesem Verfahrensschritt können auch eine Position und ein Vitalparameter von einer Vielzahl von, insbesondere von allen, Insassen des Kraftfahrzeugs 10 basierend auf einem Ergebnis der CIR-Messungen ermittelt werden. Die ermittelte Position und der ermittelte Vitalparameter werden in einer Speichereinheit des Kraftfahrzeugs 10 abgelegt.

In einem dritten Verfahrensschritt 54 wird ein Unfall des Kraftfahrzeugs 10 erfasst. Dieser kann unter Verwendung einer Sensoreinheit des Kraftfahrzeugs 10 und der Steuereinheit 12 erfolgen. Die Sensoreinheit umfasst hierfür einen Schocksensor, dessen erfassten Werte an die Steuereinheit 12 übergeben werden, wobei die Steuereinheit 12 eingerichtet ist, basierend auf den erfassten Parameterwerten des Schocksensors zu unterscheiden, ob eine Unfallsituation des Kraftfahrzeugs 10 vorliegt oder nicht, Die Sensoreinheit kann allerdings auch andere Sensoren zum Erfassen eines Unfalls des Kraftfahrzeugs 10 umfassen und die Steuereinheit 12 kann entsprechend eingerichtet sein, basierend auf den von den anderen Sensoren erfassten Werten zu entscheiden, ob ein Unfall vorliegt oder nicht.

In einem vierten Verfahrensschritt 56 wird das Funksystem zum erneuten Durchführen des auf CIR-Messungen basierenden Verfahrens unter Verwendung der ersten UWB-Antenne 14 und der zweiten UWB-Antenne 16 nach dem Unfall angesteuert. Auch in diesem Verfahrensschritt können die CIR-Messungen in beliebiger Kombination zwischen den UWB-Antennen 14, 16, 18, 20, 22 erfolgen.

In einem fünften Verfahrensschritt 58 wird die Position und der Vitalparameter des Insassen basierend auf einem Ergebnis der erneuten CIR-Messungen ermittelt. Die erneut ermittelte Position und der erneut ermittelte Vitalparameter werden ebenfalls in der Speichereinheit des Kraftfahrzeugs 10 abgelegt. Der vierte und fünfte Verfahrensschritt 56, 58 können beliebig oft, insbesondere periodisch, wiederholt werden, um den Fahrzeuginnenraum zu überwachen.

In einem sechsten Verfahrensschritt 60 werden die ermittelten Positionen und die ermittelten Vitalparameter des Insassen in Gestalt eines Notfallsignals unter Verwendung des Funksystems in die Fahrzeugumgebung ausgesendet. Dadurch, dass das Notfallsignal gezielte Informationen über die Position und den Vitalparameter vor und nach dem Unfall umfasst, können Rettungskräfte auf Basis der zusätzlichen Informationen im Notfallsignal eine gezielte und damit verbesserte Rettungsmaßnahme des oder der Insassen des Kraftfahrzeugs 10 durchführen. Dadurch können Leben besser gerettet werden und die Unfallfolgen reduziert werden.

Wie in Figur 2 exemplarisch dargestellt ist, können mittels CIR-Messungen zwischen der ersten und der zweiten UWB-Antenne 14, 16 ein oder mehrere Insassen auf den vorderen Sitzplätzen des Kraftfahrzeugs 10 sitzplatzaufgelöst erkannt werden (siehe Pfeil zwischen UWB-Antennen 14 und 16). Um allerdings auch Insassen auf anderen Sitzplätzen in dem Innenraum des Kraftfahrzeugs 10 zu ermitteln, sind die weiteren UWB-Antennen 18, 20, 22 vorgesehen. Anhand der Pfeile zwischen den UWB-Antennen 14 und 18 sowie 16 und 18 wird anschaulich, dass durch entsprechende CIR-Messungen zwischen diesen UWB-Antennen 14, 16, 18 auch Insassen auf den hinteren Sitzplätzen des Kraftfahrzeugs 10 sitzplatzaufgelöst erkannt werden können. Es ist offensichtlich, dass die genannten Vorteile auch zwischen den UWB-Antennen 14 und 22 und 16 und 24 oder - wie in Figur 3 explizit dargestellt - zwischen den UWB-Antennen 18, 20, 22 im hinteren Fahrzeugteil entsprechend für die hinteren Sitzplätze erzielt werden.

In den Figuren 2 und 3 ist ein weiterer Aspekt dargestellt, wonach die UWB-Antennen 14, 16, 18, 20, 22 nicht nur für gegenseitige CIR-Messungen verwendet werden können. Vielmehr kann jede UWB-Antenne 14, 16, 18, 20, 22 für sich genommen UWB-Pulse aussenden und die Echosignale des Fahrzeuginnenraums anhand von empfangenen Impulsantworten empfangen. Aus diesen empfangenen Impulsantworten können ebenfalls die Position und die Vitalparameter des oder der Insassen ermittelt werden. Beispielshaft ist eine Reichweite des Verfahrens der empfangenen Impulsantworten jeder UWB-Antenne 14, 16, 18, 20, 22 in Gestalt eines gestrichelten Kreises dargestellt. Die Kreise, die in etwa einen Radius von einem Meter aufweisen, sind allerdings nicht auf diese Maßangabe beschränkt. Vielmehr können mit diesem Verfahren auch Reichweiten von bis zu 10 Meter von der jeweiligen UWB-Antenne 14, 16, 18, 20, 22 erzielt werden. Insofern kann das hier vorgestellte Verfahren auch auf Kraftfahrzeuge 10 mit größeren Fahrzeuginnenräumen wie Transportern, Bussen, Wohnwagen und so weiter Anwendung finden. Anschaulich ist in Figuren 2 und 3 gezeigt, dass mit den exemplarisch fünf gewählten UWB-Antennen 14, 16, 18, 20, 22 der gesamte Innenraum des Kraftfahrzeugs 10 abgetastet und somit überwacht werden kann. Folglich können die Position und der Vitalparameter eines jeden Insassen des Kraftfahrzeugs 10 ermittelt werden.

Ebenfalls in den Figuren 2 und 3 ist illustriert, dass der Sendeempfänger des Funksystems ferner eine erste und eine zweite NFC-Antenne 24, 26 aufweist. Die erste NFC-Antenne 24 ist in der Fahrertür angeordnet, während die zweiten NFC-Antenne 26 in der Tür hinter der Beifahrertür eingebaut ist. Die NFC-Antennen 24, 26 können verwendet werden, um mittels NFC die ermittelten Positionen und die ermittelten Vitalparameter des oder der Insassen des Kraftfahrzeugs 10 vor und nach dem Unfall an Rettungskräfte zu übermitteln. Diese beispielhafte Positionierung hat den Vorteil, dass das Kraftfahrzeugs 10 von zwei gegenüberliegenden Seiten aus eine Kommunikation mittels NFC ermöglicht, sodass in den meisten Unfallsituationen mit zumindest einer der beiden NFC-Antennen 24, 26 eine Kommunikation aufgebaut werden kann. In einem Speicher der jeweiligen NFC-Antenne 24, 26 sind die ermittelten Positionen und Vitalparameter des oder der Insassen des Kraftfahrzeugs 10 vor und nach dem Unfall abgelegt. Vorteilhaft können Rettungskräfte mittels einer NFC-Kommunikation an diese Informationen gelangen und individuell geeignete Rettungsmaßnahmen durchführen. Rein exemplarisch ist die erste NFC-Antenne 24 als NFC-Reader und die zweite NFC-Antenne 26 als NFC-Tag ausgestaltet. Dies hat den Vorteil, dass selbst bei einer zusammengebrochenen Stromversorgung des Kraftfahrzeugs 10 die Informationen über die zweite NFC-Antenne 26 von den Rettungskräften empfangen werden können.

### Bezugszeichenliste

- 10: Kraftfahrzeug
- 12: Steuereinheit
- 14: erste UWB-Antenne
- 16: zweite UWB-Antenne
- 18: dritte UWB-Antenne
- 20: vierte UWB-Antenne
- 22: fünfte UWB-Antenne
- 24: erste NFC-Antenne
- 26: zweite NFC-Antenne
- 50: erster Verfahrensschritt
- 52: zweiter Verfahrensschritt
- 54: dritter Verfahrensschritt
- 56: vierter Verfahrensschritt
- 58: fünfter Verfahrensschritt
- 60: sechster Verfahrensschritt

## Patentansprüche

1. Computerimplementiertes Verfahren zum Ermitteln von Position und Vitalparameter eines Insassen eines Kraftfahrzeugs (10), wobei das Kraftfahrzeug (10) ein Funksystem mit einem Sendeempfänger, mit einer ersten UWB-Antenne (14) und mit einer zweiten UWB-Antenne (16) und eine mit dem Funksystem verbundene Steuereinheit (12) aufweist, das Verfahren umfassend die folgenden Schritte:
- Ansteuern des Funksystems zum Durchführen eines auf Channel Impulse Response-(CIR-)Messungen basierenden Verfahrens unter Verwendung der ersten UWB-Antenne (14) und der zweiten UWB-Antenne (16) während des Betriebs des Kraftahrzeugs (10),
- Ermitteln der Position und eines Vitalparameters des Insassen basierend auf einem Ergebnis der CIR-Messungen,
- Erfassen eines Unfalls des Kraftfahrzeugs (10),
- Ansteuern des Funksystems zum erneuten Durchführen des auf CIR-Messungen basierenden Verfahrens zum Abtasten des Innen- und Außenraums des Kraftfahrzeugs (10) unter Verwendung der ersten UWB-Antenne (14) und der zweiten UWB-Antenne (16) nach dem Unfall,
- Ermitteln der Position und des Vitalparameters des Insassen basierend auf einem Ergebnis der erneuten CIR-Messungen,
- Vergleichen der ermittelten Position und/oder des Vitalparameters des Insassen vor dem Unfall mit der ermittelten Position und/oder dem Vitalparameter des Insassen nach dem Unfall, um festzustellen, ob der Insasse durch den Unfall aus dem Kraftfahrzeug (10) geschleudert wurde.

2. Verfahren nach Anspruch 1, wobei eine sitzplatzaufgelöste Erkennung des Insassen des Kraftfahrzeugs (10) basierend auf der ermittelten Position des Insassen vor und nach dem Unfall erfolgt.

3. Verfahren nach Anspruch 1, wobei das Funksystem basierend auf einem Ergebnis des Vergleichens angesteuert wird, ein Notfallsignal in die Fahrzeugumgebung auszusenden, wobei das Notfallsignal die ermittelten Positionen und/oder die ermittelten Vitalparameter des Insassen vor und nach dem Unfall umfassen.

4. Verfahren nach Anspruch 3, wobei die erste UWB-Antenne (14) und die zweite UWB-Antenne (16) ferner zum Senden und Empfangen von Bluetooth-Funksignalen eingerichtet sind und das Funksystem angesteuert wird, das Notfallsignal als UWB- und/oder Bluetooth-Funksignal unter Verwendung der ersten UWB-Antenne (14) und/oder zweiten Antenne (16) auszusenden.

5. Verfahren nach Anspruch 3 oder 4, wobei das Notfallsignal allgemeine Informationen über das Kraftfahrzeug (10) umfasst.

6. Verfahren nach einem der Ansprüche 3 bis5, wobei das Funksystem ferner eine NFC-Antenne (24, 26) zum Übertragen des Notfallsignals umfasst, wobei nach einem Unfall die für das Notfallsignal erforderlichen Informationen in einem Speicher hinterlegt werden.

7. Verfahren nach einem der vorherigen Ansprüche, ferner aufweisend folgende Schritte:
- Ansteuern des Funksystems zum Senden von UWB-Pulsen und Empfangen von Impulsantworten unter Verwendung von mindestens einer der beiden UWB-Antennen (14, 16) und
- Ermitteln der Position und/oder des Vitalparameters des Insassen basierend auf einem Ergebnis der empfangenden Impulsantworten.

8. Kraftfahrzeug (10), aufweisend:
- ein Funksystem mit einem Sendeempfänger, mit einer ersten UWB-Antenne (14) und einer zweiten UWB-Antenne (16) und
- eine mit dem Funksystem verbundene Steuereinheit (12), die eingerichtet ist, das Verfahren nach einem der vorangehenden Ansprüche durchzuführen.

## Claims

1. Computer-implemented method for determining the position and a vital parameter of an occupant of a motor vehicle (10), wherein the motor vehicle (10) has a radio system having a transceiver, having a first UWB antenna (14) and having a second UWB antenna (16), and has a control unit (12) which is connected to the radio system, the method comprising the following steps:
- controlling the radio system to perform a channel impulse response (CIR) measurement-based method using the first UWB antenna (14) and the second UWB antenna (16) during operation of the motor vehicle (10),
- determining the position and a vital parameter of the occupant on the basis of a result of the CIR measurements,
- detecting an accident of the motor vehicle (10),
- controlling the radio system to repeat the CIR measurement-based method to scan the interior and exterior of the motor vehicle (10) using the first UWB antenna (14) and the second UWB antenna (16) after the accident,
- determining the position and the vital parameter of the occupant on the basis of a result of the repeated CIR measurements,
- comparing the determined position and/or the vital parameter of the occupant before the accident with the determined position and/or the vital parameter of the occupant after the accident in order to determine whether the occupant was thrown from the motor vehicle (10) as a result of the accident.

2. Method according to claim 1, wherein seat-resolved detection of the occupant of the motor vehicle (10) is carried out on the basis of the determined position of the occupant before and after the accident.

3. Method according to claim 1, wherein the radio system is controlled, on the basis of a result of the comparison, to emit an emergency signal into the vehicle environment, wherein the emergency signal comprise the determined positions and/or the determined vital parameters of the occupant before and after the accident.

4. Method according to claim 3, wherein the first UWB antenna (14) and the second UWB antenna (16) are further designed to send and receive Bluetooth radio signals and the radio system is controlled to emit the emergency signal as a UWB and/or Bluetooth radio signal using the first UWB antenna (14) and/or second antenna (16).

5. Method according to claim 3 or 4, wherein the emergency signal comprises general information regarding the motor vehicle (10).

6. Method according to any of claims 3 to 5, wherein the radio system further comprises an NFC antenna (24, 26) for transmitting the emergency signal, wherein after an accident, the information required for the emergency signal is stored in a memory.

7. Method according to any of the preceding claims, further comprising the following steps:
- controlling the radio system to send UWB pulses and receive impulse responses using at least one of the two UWB antennas (14, 16) and
- determining the position and/or the vital parameter of the occupant on the basis of a result of the receiving impulse responses.

8. Motor vehicle (10) comprising:
- a radio system having a transceiver, having a first UWB antenna (14) and a second UWB antenna (16) and
- a control unit (12) which is connected to the radio system and designed to perform the method according to any of the preceding claims.

## Revendications

1. Procédé mis en œuvre par ordinateur pour la détermination de la position et des paramètres vitaux d'un occupant d'un véhicule automobile (10), dans lequel le véhicule automobile (10) présente un système radio comportant un émetteur-récepteur, comportant une première antenne ULB (14) et comportant une seconde antenne ULB (16), et une unité de commande (12) connectée au système radio, le procédé comprenant les étapes suivantes :
- commande du système radio pour la réalisation d'un procédé basé sur des mesures de réponse impulsionnelle de canal (CIR) à l'aide de la première antenne ULB (14) et de la seconde antenne ULB (16) pendant le fonctionnement du véhicule automobile (10),
- détermination de la position et d'un paramètre vital de l'occupant à partir d'un résultat des mesures CIR,
- détection d'un accident du véhicule automobile (10),
- commande du système radio pour la réalisation de nouveau du procédé basé sur des mesures CIR pour balayer l'intérieur et l'extérieur du véhicule automobile (10) à l'aide de la première antenne ULB (14) et de la seconde antenne ULB (16) après l'accident,
- détermination de la position et des paramètres vitaux de l'occupant sur la base d'un résultat des nouvelles mesures CIR,
- comparaison de la position et/ou du paramètre vital de l'occupant déterminés avant l'accident avec la position et/ou le paramètre vital de l'occupant déterminés après l'accident, afin de définir si l'occupant a été éjecté du véhicule automobile (10) à la suite de l'accident.

2. Procédé selon la revendication 1, dans lequel une reconnaissance de l'occupant du véhicule automobile (10) différenciée selon les places assises est effectuée sur la base de la position de l'occupant déterminée avant et après l'accident.

3. Procédé selon la revendication 1, dans lequel le système radio est commandé, sur la base d'un résultat de la comparaison, pour émettre un signal d'urgence dans l'environnement du véhicule, dans lequel le signal d'urgence comprend les positions déterminées et/ou les paramètres vitaux déterminés de l'occupant avant et après l'accident.

4. Procédé selon la revendication 3, dans lequel la première antenne ULB (14) et la seconde antenne ULB (16) sont en outre configurées pour émettre et recevoir des signaux radio Bluetooth, et le système radio est commandé pour émettre le signal d'urgence sous forme de signal radio ULB et/ou Bluetooth à l'aide de la première antenne ULB (14) et/ou de la seconde antenne (16).

5. Procédé selon la revendication 3 ou 4, dans lequel le signal d'urgence comprend des informations générales concernant le véhicule automobile (10).

6. Procédé selon l'une des revendications 3 à 5, dans lequel le système radio comprend en outre une antenne NFC (24, 26) destinée à transmettre le signal d'urgence, dans lequel les informations nécessaires au signal d'urgence sont enregistrées dans une mémoire après un accident.

7. Procédé selon l'une des revendications précédentes, présentant en outre les étapes suivantes :
- commande du système radio pour émettre des impulsions ULB et recevoir des réponses impulsionnelles à l'aide d'au moins l'une des deux antennes ULB (14, 16) et
- détermination de la position et/ou du paramètre vital de l'occupant sur la base d'un résultat des réponses impulsionnelles reçues.

8. Véhicule automobile (10), présentant :
- un système radio comportant un émetteur-récepteur, comportant une première antenne ULB (14) et une seconde antenne ULB (16), et
- une unité de commande (12) connectée au système radio et configurée pour réaliser le procédé selon l'une des revendications précédentes.
